# EUROPEAN PATENT SPECIFICATION

(11) **EP 0 770 384 B1**
(45) Date of publication and mention of the grant of the patent: **06.02.2002**
(21) Application number: 96116870.5
(22) Date of filing: 21.10.1996
(51) Int. Cl.: A61K 9/10, A61K 9/02, A61K 9/48

(54) **Solid, anhydrous, pharmaceutical compositions for vaginal use**
Feste, wasserfreie, pharmazeutische Zubereitungen für vaginale Verwendung
Compositions solides, anhydres et pharmaceutiques pour utilisation vaginale

(30) Priority: 27.10.1995 IT MI952221
(43) Date of publication of application: 02.05.1997
(73) Proprietor: MonteResearch S.r.l., 20124 Milano (IT)
(72) Inventor: Ronchi, Celestino, 20016 Pero (IT); Berlati, Fabio, 20016 Pero (IT); Scaglione, Francesco, 20016 Pero (IT)
(74) Representative: Minoja, Fabrizio, Dr.

(56) References cited:
- EP-A- 0 153 836
- FR-A- 2 335 203
- US-A- 4 292 299
- US-A- 4 853 211
- US-A- 5 369 131

## Description

### Filed of the Invention

The present invention relates to pharmaceutical compositions for vaginal use.

### Background of the Invention

Bioadhesive polymers have been and still are the subject of in-depth studies. Bioadhesive polymers are generally known for their ability of being bound to biological membranes.

Numerous patents have come into being which have as their object the use of muco-adhesives in the form of tablets which contain a bioadhesive layer and a non-adhesive layer containing the medicament (U.S. Patent No. 4,292,299 to the Suzuki, et al.) or controlled release treatment compositions which, when in the presence of sufficient water to swell the bioadhesive, will adhere to skin or to a mucosal membrane (U.S. Patent No. 4,615,697 to J.R. Robinson). Other patents have been developed for muco-adhesive compositions which will bioadhesively attach to a mucosal membrane, but ca be removed by tap water if one wishes. Wang et al. (EP 93305950.3) disclosed the application of polyvinyl pyrrolidone-based muco-adhesive polymers and carboxy-functional polymers (characterized by the presence of carboxyl groups in the chains forming the polymer) dispersed in a pharmaceutically acceptable vehicle, preferably water, in an amount sufficient to form a liquid suspension, preferably a gel.

Moreover, products containing a water-insoluble bioadhesive polymer and formulated as an oil-in-water emulsion for the treatment of vaginal dryness are commercially available (REPLENS- by Warner-Lambert Co.).

Compositions containing bioadhesive polymers and therapeutic agents provide the desired contact between the mucosal membrane and the composition. The composition is left in contact with the mucosal membrane for a sufficient time for the treating agent to be released over a controlled period.

It has now been found that an anhydrous solid pharmaceutical composition employing polycarbophil as the sole muco-adhesive polymer dispersed in a pharmaceutically acceptable vehicle improves residence at the application site and provides a long-lasting effetc.

Pharmaceutical forms employing water-insoluble mucoadhesive agents containing water are already hydrated and immediately bond to a mucosal membrane upon application. Once applied, the composition is left in contact with the mucosal membrane for a sufficient time for the treating agent to be released over a controlled period, allowing for local or systemic activity.

On the other hand, anhydrous pharmaceutical compositions containing mucoadhesive agents are hydrated only after contact with the mucosa. When the preparation is applied to the vaginal mucosa, the pharmaceutical form simultaneously disintegrates, forming an adhesive layer on the mucosa containing the active ingredient, allowing for local or systemic activity.

### Description of the Invention

The present invention relates to anhydrous solid pharmaceutical compositions for vaginal use with at least one active ingredient comprising polycarbophil as the sole muco-adhesive polymer dispersed in a pharmaceutically acceptable vehicle. In addition, the composition may also contain pharmaceutically acceptable additives. These additives include stabilizers, preservatives, excipients, binders, coloring agents, odor controlling agents and the like.

Polycarbophil is a muco-adhesive polymer well known to those skilled in the art. The content of polycarbophil in the composition of the invention ranges from 0.5% to 90% by weight of the composition.

The pharmaceutically acceptable vehicle is the bulk substance through which the bioadhesive polymer and the active ingredients (s) are distributed to form a dispersion. This vehicle must also be capable of being used in or on humans or other mammals without causing any ill effects, such as toxicity or severe irritation to the mucosal tissue. Pharmaceutically acceptable vehicles suitable for use in the present invention include powders, solid and semi-solid lipid masses, and anhydrous liquids, normally used in pharmaceutical and cosmetic preparations.

The active ingredients suitable for use in the present invention are of different types (natural, semisynthetic and synthetic) and include antimycotic agents, antiprotozoal agents, disinfectants, hormones, antibiotics, chemotherapeutic agents and others) and are generally present in the composition ranging from 0,002% to 90% by weight of the composition.

Examples of active ingredients used in the present invention are imidazole antimycotics such as miconazole, econazole, ketonazole, local antiseptics and hormones.

The composition of the present invention may take the form of vaginal tablets, soft gelatin capsules, semi-synthetic glyceride-based suppositories, which may or may not contain surfactant substances and polyethylene glycol-based suppositories.

The dosage forms of the invention may be prepared by experts of the art according to well known techniques (see Remington's Pharmaceutical Sciences Handbook, XVII ed., Mack Pub., N.Y., U.S.A.).

A study was carried out on a total of 20 healthy female volunteers in order to compare the release rates of econazole nitrate from a conventional drug without a bioadhesive and from three formulations, each containing a different concentration of polycarbophil as mucoadhesive agent (25, 50, and 100 mg/suppository). Vaginal fluid samples were taken at 0 and at 2, 6, 12 and 24 h from administration and drug concentrations measured by HPLC. Results showed slower release rates of econazole nitrate plus the muco-adhesive as compared to the commercially available drug. High vaginal fluid drug concentrations were measured up to 24 h following the administration of the vaginal suppositories containing polycarbophil while no drug was detected 12 h following the administration of the commercially available product.

**Table:**

| Econazole levels (mg/ml) in vaginal fluid | | | | | |
|---|---|---|---|---|---|
| Product Time (hours) | 0 | 2 | 6 | 12 | 24 |
| Commercial | | | | | |
| Product | 0 | 48.6 | 0.85 | 0 | 0 |
| Formulation 1 | 0 | 56.4 | 44.7 | 36.9 | 0.12 |
| Formulation 2 | 0 | 88.7 | 54.3 | 28.9 | 12.6 |
| Formulation 3 | 0 | 96.8 | 68.3 | 46.3 | 18.9 |

The sustained release of the drug is regulated by the presence of the muco-adhesive polymer.

The use of polycarbophil in the manufacture of anhydrous solid vaginal pharmaceutical compositions with controlled release fall within the scope of the present invention.

The following examples further illustrate the invention.

### Example No. 1: Vaginal suppositories containing an antimycotic Econazole Nitrate (active ingredient)

| Component | Unitary Formula |
|---|---|
| Econazole Nitrate | 100 mg |
| Policarbophyl (Noveon® AA1) | 100 mg |
| Semi-synthetic Triglicerides (m.p. 37°C) | q.s. to 3 g |

### Example 2: Vaginal suppositories containing an antimycotic Miconazole Nitrate (active ingredient)

| Component | Unitary Formula |
|---|---|
| Miconazole Nitrate | 100 mg |
| Policarbophyl (Noveon® AA1) | 200 mg |
| Polyethylene-glycol mixture | q.s. to 3 g |

### Example 3: Vaginal suppositories containing Povidone-Iodine (active ingredient)

| Component | Unitary Formula |
|---|---|
| Povidone-Iodine (Iodine 10%) | 200 mg |
| Policarbophyl (Noveon® AA1) | 200 mg |
| Polyethylene-glycol mixture | q.s. to 3 g |

### Example N. 4: Soft-gelatine capsules containing Estriol (active ingredient)

| Component | Unitary Formula |
|---|---|
| Estriol | 0.5 mg |
| Policarbophyl (Noveon® AA1) | 50 mg |
| Labrafil® | q.s. to 500 mg |
| Gelatin shell | q.s. |

## Claims

1. An anhydrous solid pharmaceutical composition for vaginal use with at least one active ingredient comprising polycarbophil as the sole mucoadhesive polymer dispersed in a pharmaceutically acceptable vehicle.

2. A composition according to claim 1, wherein the amount of polycarbophil is from 0.5% to 90% by weight of the composition.

3. A composition according to claims 1-2, wherein the active ingredient(s) are selected from the group consisting of antimycotic agents, antiprotozoal agents, disinfectants, hormones, antibiotics, chemotherapeutic agents.

4. A composition according to claims 1-3, in form of vaginal tablets, soft gelatin capsules, semi-synthetic glyceride-based suppositories, optionally containing surfactant substances and polyethylene glycol-based suppositories.

5. Use of polycarbophil in the manufacture of controlled release, anhydrous, solid pharmaceutical compositions.

## Patentansprüche

1. Wasserfreie pharmazeutische Feststoff-Zusammensetzung zur vaginalen Verwendung mit mindestens einer Wirksubstanz, die Polycarbophil als das einzige an Schleimhäuten haftfähige Polymer in einem pharmazeutisch annehmbaren Träger dispergiert umfasst.

2. Zusammensetzung nach Anspruch 1, wobei die Menge an Polycarbophil 0,5 Gew.-% bis 90 Gew.-% der Zusammensetzung ist.

3. Zusammensetzung nach Anspruch 1 oder Anspruch 2, wobei die Wirkstoffsubstanz (die Wirkstoffsubstanzen) aus der Gruppe bestehend aus antimykotischen Mitteln, Mitteln gegen Protozoon, Desinfektionsmitteln, Hormonen, Antibiotika, chemotherapeutischen Mitteln ausgewählt ist (sind).

4. Zusammensetzung nach einem der Ansprüche 1 bis 3 in Form von vaginalen Tabletten, weichen Gelatinekapseln, Suppositorien auf der Basis halbsynthetischer Glyceride und Suppositorien auf Polyethylenbasis.

5. Verwendung von Polycarbophil bei der Herstellung von wasserfreien pharmazeutischen Feststoff-Zusammensetzungen mit kontrollierter Freisetzung.

## Revendications

1. Composition pharmaceutique solide anhydre à usage vaginal contenant au moins un principe actif comprenant du polycarbophile en tant que seul polymère mucoadhésif disposé dans un véhicule pharmaceutiquement acceptable.

2. Composition selon la revendication 1, dans laquelle la quantité de polycarbophile est de 0,5 à 90% en poids de la composition.

3. Composition selon les revendications 1-2, dans laquelle le(s) principe(s) actif(s) est(sont) sélectionné(s) dans le groupe constitué par les agents antimycotiques, les agents antiprotozoaires, les désinfectants, les hormones, les antibiotiques et les agents chimiothérapeutiques.

4. Composition selon les revendications 1-3, sous forme de comprimés vaginaux, de capsules de gélatine molle, de suppositoires à base de glycéride semi-synthétique contenant éventuellement des substances tensio-actives et de suppositoires à base de polyéthylène glycol.

5. Utilisation de polycarbophile dans la fabrication de compositions pharmaceutiques solides, anhydres et à libération contrôlée.
